(19) Europäisches Patentamt
European Patent Office
Office européen des brevets

(11) EP 1 476 171 B1

(12) **EUROPEAN PATENT SPECIFICATION**

(45) Date of publication and mention
of the grant of the patent:
**08.09.2010 Bulletin 2010/36**

(51) Int Cl.:
***A61K 33/00*** *(2006.01)*

(21) Application number: **01959904.2**

(22) Date of filing: **19.07.2001**

(86) International application number:
**PCT/ZA2001/000100**

(87) International publication number:
**WO 2002/005849 (24.01.2002 Gazette 2002/04)**

(54) **TRANSPORTATION OF NUCLEIC ACID SUBSTANCES**

TRANSPORT VON NUKLEINSÄURESUBSTANZEN

TRANSPORT DE SUBSTANCES D'ACIDES NUCLEIQUES

(84) Designated Contracting States:
**AT BE CH CY DE DK ES FI FR GB GR IE IT LI LU MC NL PT SE TR**

(30) Priority: **19.07.2000 ZA 200003643**

(43) Date of publication of application:
**17.11.2004 Bulletin 2004/47**

(73) Proprietor: **North-West University Potchefstroom 2520 (ZA)**

(72) Inventor: **MEYER, Petrus, Johannes**
**6573 Sedgefield (ZA)**

(74) Representative: **Bates, Philip Ian et al**
**Reddie & Grose**
**16 Theobalds Road**
**London**
**WC1X 8PL (GB)**

(56) References cited:
**WO-A-93/25213      WO-A-97/17978**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

This invention relates to preparations for use in transporting nucleic acid substances to and into cells of a plant or an animal body (which expression is herein intended to include the human body), either *in vivo* or *in vitro, or of a micro-organism*. This invention is more particularly concerned with the administration of substances based on nucleic acid to patients for the purpose of the transportation thereof through the body of the patient and the ultimate delivery thereof into cells either generally or of a targeted cell type. It therefore finds application in what is colloquially referred to as gene therapy as will become evident from the disclosure below. The invention is also concerned with the transfection of plants and micro-organisms with DNA thereby to affect the genetic properties of such plant or organism.

**BACKGROUND TO THE INVENTION**

The concept of introducing heterologous genetic material into the cells of an organism for the purpose of allowing such genetic material to be incorporated into the cellular DNA of such organism is well known. It is now routinely exercised *in vitro* and various methods have been developed for the introduction of such heterologous genetic material into the cells of an organism.

The most established of these methods is commonly referred to as the gene gun technique. By this technique genetic material is quite literally shot into the cells of the organism. While some of the cells do not survive the blast, those that do survive are caused to proliferate. Some of these proliferating survivors will have incorporated into its own genome the genetic material shot into it in the form of the DNA. The gene so incorporated may eventually be expressed to yield the product coded for by the DNA so introduced into the organism. It is also known to make use of other forms of vectors by which a gene which is required to be expressed is introduced into an organism. Such vectors include viruses.

While these techniques work adequately in the appropriate *in vitro* environment on lower organisms such as bacteria, they are not regarded as being generally suitable for implementation *in vivo* for the introduction of genetic material into a living animal, such as man. The harshness of the gene gun technique by its very nature renders that technique generally unsuitable for in vivo application. It is further generally regarded to be advisable not to expose animals or human beings unnecessarily to viral infections, let alone where such infections are by transgenic viruses, the full genetic nature and potential for mutation of which may be unknown.

There has thus been a long-felt need for an appropriate process by which genetic material may be introduced into selected cells there to express and yield a desired medicinally active substance.

This need is sought to be addressed, inter alia by the disclosure contained in US patent 6,258,789 (German, et al.) and the patents cited during the examination thereof. German et al discloses a method of delivering a secreted protein into the bloodstream of a mammalian subject by introducing into the gastro-intestinal tract of a mammalian subject, by oral administration, a construct comprising a nucleic acid molecule encoding the desired secreted protein and a promoter sequence operably linked to the nucleic acid molecule, wherein said construct is not packaged in a viral particle, and wherein the method involves introducing the nucleic acid construct into the intestinal epithelial cells of the animal in question so that secretion of the protein takes place at that locus to be available for absorption into the body from the digestive tract.

In EP93912877.3 and US patent 5,633,284 and their equivalents the applicant disclosed that dermatological or topical compositions comprising the combination of nitrous oxide [$N_2O$] and at least one fatty acid, or lower alkyl ester thereof, in a dermatologically acceptable carrier medium, are useful in the treatment of a variety of skin, muscle and joint disorders. It further disclosed therein that such combinations may beneficially also include additional active ingredients. The following active ingredients are specifically mentioned in this regard: coal tar solution, collagen, nicotinamide, nicotinic acid, lanolin, vitamin E, methyl salicylate, arnica and H1-antagonist antihistamines of which only diphenhydramine chloride is specifically mentioned.

No agent based on nucleic acid was amongst the active ingredients specifically mentioned in these patents.

In WO97/17978 and US patent 6,221,377 and their corresponding applications and patents the present applicant disclosed that the action of analgesic, anti-inflammatory and antipyretic drugs may be enhanced by administering such drugs in conjunction with a medium which comprises nitrous oxide and at least one long chain fatty acid selected from the group consisting of oleic acid, linoleic acid, alpha-linolenic acid, gamma linolenic acid, arachidonic acid, and any of the $C_1$ to $C_6$ alkyl esters of such long chain fatty acids, mixtures of such acids and mixtures of such esters. The medium may comprise the mixture known as Vitamin F Ethyl Ester and may optionally further comprise eicosapentaenoic acid [$C20:5\omega3$] and decosahexaenoic acid [$C22:6\omega3$].

While these applications do disclose a systemic effect in that the formulations are, for example, topically applied or orally administered to have an effect, say, in an affected joint or muscle, the specification again fails to disclose the mode or route of transport of the active ingredient from its site of administration to its locus of effect, and speculation in this regard would probably be based on *ex post facto* hindsight.

It has now surprisingly been found that the aforesaid medium and other similar media have the unexpected property that it may also be used as a delivery vehicle adapted to be used for the delivery, into the nucleus of, or generally to, an animal cell, of nucleic acid compounds.

It has been pointed out in WO97/17978 referred to above that nitrous oxide is a natural gas which is also produced synthetically, that it is also known by the trivial name "laughing gas", and that it has been in use for many years as an inhalation anaesthetic and analgesic, particularly in dentistry.

It was further stated that nitrous oxide has been reported to have a synergistic or potentiating effect on halothane and other gaseous anaesthetics [See Goodman & Gilman's The Pharmacological Basis of Therapeutics 8th Ed. 1990 pp. 298-300].

Since such known synergism or potentiation is based on the use of nitrous oxide administered by inhalation, and since the use of nitrous oxide on its own as an anaesthetic and analgesic has likewise been in the form of an inhalation agent, the use of nitrous oxide for all these purposes has been confined to hospitalised patients or, at best, to treatments carried out by medical practitioners in their consulting rooms, or treatments carried out by or under supervision of a nurse in charge of a home-care patient.

Nitrous oxide is known to be soluble in water and it has been reported that at 20°C and 2 atm pressure one litre of the gas dissolves in 1,5 litres of water, see The Merck Index 10th Ed. p. 6499.

Nitrous oxide is also known for its use as a propellant gas, mainly as a substitute for propellant gases such as chlorofluorocarbons, and more particularly to produce a food product mousse such as whipped cream or chocolate mousse or quick-breaking foams for hair treatment preparations. See in this regard U.K. Patent 1033299, U.K. Patent 1105919 and European Patent Application EPA-0123827. None of these prior publications suggests that the nitrous oxide gas play any other role than a physical one, i.e. to expand on being depressurised and thereby to create a mousse or a foam. In fact it is typically regarded as an inert In these applications and useful due to the fact that it is colourless, odourless and tasteless but soluble in water and oils.

There appears to be no suggestion in the literature, other than the applicant's own prior patents and patent applications referred to above, that aqueous solutions of nitrous oxide might have any effect on man or animals. As far as the present inventors know it has also never been suggested that nitrous oxide may be used in conjunction with any nucleic acid substance for any purpose, and In particular in connection with the transportation thereof In the human or animal body.

**OBJECT OF THE INVENTION**

It is an object of the present invention to provide a method and formulation for the transportation and delivery of nucleic acid substances into cells of an animal, a plant or a micro-organism. The term "animal," is herein intended to be interpreted In its wide meaning to include man and insect. The term "micro-organlsm" is herein intended to Include single and multi-cellular organisms such as parasites. The invention is thus concerned with all forms of life in which nucleic acid substances in the form of DNA or RNA determine the genetic properties of that form of life.

**STATEMENTS OF THE INVENTION**

According to the present invention there is provided a method for the administration of a nucleic acid substance to the cells of a plant or a micro-organism or for the in vitro administration of a nucleic acid substance to the cells of an animal, the method being **characterised in that** the nucleic acid substance is formulated with an administration medium which comprises a solution of nitrous oxide gas in a physiologically acceptable carrier solvent for the gas and which administration medium includes at least one fatty acid or ester or other suitable derivative thereof selected from the group consisting of oleic acid, linoleic acid, alpha-linolenic acid, gamma-linolenic acid, arachidonic acid, eicosapentaenoic acid [C20: $5\omega3$], decosahexaenoic acid [C22: $6\omega3$], ricinoleic acid and derivatives thereof selected from the group consisting of the C1 to C6 alkyl esters thereof, the glycerol-polyethylene glycol esters thereof and the reaction product of hydrogenated natural oils composed largely of ricinoleic acid based oils, such as castor oil, with ethylene oxide.

According to a further aspect of the present invention there is provided a preparation for use in introducing a nucleic acid substance into the cells of an animal, a plant or a micro-organism **characterised in that** the preparation comprises a formulation of the nucleic acid in an administration medium which comprises a solution of nitrous oxide gas in a physiologically acceptable carrier solvent for the gas and which administration medium includes at least one fatty acid or ester or other suitable derivative thereof selected from the group consisting of oleic acid, linoleic acid, alpha-linolenic acid, gamma-linolenlc acid, arachidonic acid, eicosapentaenoic acid [C20: $5\omega3$], decosahexaenoic acid [C22: $6\omega3$], ricinoleic acid and derivatives thereof selected from the group consisting of the C1 to C6 alkyl esters thereof, the glycerol-polyethylene glycol esters thereof and the reaction product of hydrogenated natural oils composed largely of ricinoleic acid based oils, such as castor oil with ethylene oxide.

The administration medium preferably includes the eicosapentaenoic acid [C20: $5\omega3$] and/or decosahexaenoic acid [C22: $6\omega3$] as additional long chain fatty acids to at least one of the other components of the carrier medium defined above.

The reaction product of hydrogenated natural oils composed largely of ricinoleic acid based oils with ethylene oxide is preferably produced from castor oil of which the fatty acid content is known to be predominantly composed of ricinoleic acid. This product is known as PEG-n-Hydrogenated Castor Oil. A range of such products is marketed by BASF under the trade description of Cremaphor RH grades. Glycerol-polyethylene glycol ester of ricinoleic acid is also marketed by the same company but under the trade description of Cremaphor EL.

The carrier solvent for the nitrous oxide gas may be water or any of the pharmaceutically acceptable alcohols, ethers, oils or polymers such as a polyethyleneglycol or the like. The oil may be organic or mineral oil. The organic oil may be an essential oil based on long chain fatty acids having between 14 and 22 carbon atoms in the fatty acid. The oil may also be of either natural or synthetic origin and, if of natural origin, it may be either plant oil or animal oil. As plant oils those rich in gamma linolenic acid [GLA] are preferred and as animal oil dairy cream may be used.

In the preferred form of the invention the solution is an aqueous solution saturated with nitrous oxide. Preferably the water is deionised and purified to be free of microbes.

When the formulation containing the nucleic acid substance to be enhanced by means of the nitrous oxide is to be in a liquid (including an encapsulated liquid) presentation for oral administration or in a nasal or bronchial or pulmonary spray or in the form of an injectable formulation, such formulation may incorporate, as part of the administration medium, water or acceptable other liquid into which the nitrous oxide is dissolved and in which the fatty acid or ester thereof is either dissolved or suspended or emulsified along with the nucleic acid substance to be enhanced by being formulated therewith.

Likewise, where the nucleic acid substance is to be administered to the patient as a topical, buccal or vaginal cream or ointment, or as a suppository, the formulation used in making up such cream, ointment, or suppository may incorporate, along with the nucleic acid substance to be enhanced, a quantity of water or other liquid containing, and preferably saturated with, nitrous oxide, the long chain fatty acid or ester thereof and the nucleic acid substance formulated therewith, and, further, such additional excipients and carriers as are conventionally used in the pharmaceutical trade in making up such dosage forms.

The carrier solvent for the nitrous oxide gas may thus in an alternative formulation according to the invention be essentially non-aqueous and composed of the least one fatty acid or ester thereof selected from the group consisting of oleic acid, linoleic acid, alpha-linolenic acid, gamma-linolenic acid, arachidonic acid, eicosapentaenoic acid [C20: $5\omega3$], decosahexaenoic acid [C22: $6\omega3$], ricinoleic acid and derivatives thereof selected from the group consisting of the C1 to C6 alkyl esters thereof, the glycerol-polyethylene glycol esters thereof and the reaction product of hydrogenated natural oils composed largely of ricinoleic acid based oils with ethylene oxide, required to be part of the formulation.

A formulation suited to transdermal application whether as an ointment, cream or lotion or in the form of a skin patch providing a reservoir for the formulation is also a preferred form of the formulation according to the invention.

The essential fatty acid, or ester thereof, component of the composition preferably comprises a mixture of esters of the fatty acids listed above. Thus, in the most preferred form of the invention the fatty acid component of the composition is constituted by the complex known as Vitamin F and in this regard it is preferred to make use of the ester form of Vitamin F known as Vitamin F Ethyl Ester. This product is commercially available under the trade description of Vitamin F Ethyl Ester CLR 110 000 Sh.L. U./g from CLR Chemicals Laboratorium Dr.Kurt Richter GmbH of Berlin, Germany. The typical fatty acid distribution of this product is as follows:

$< C_{16}$ : O
$C_{16.0}$ : 8,3 %
$C_{18.0}$ : 3,5 %
$C_{18.1}$ : 21,7 %
$C_{18.2}$ : 34,8 %
$C_{18.4}$ : 28,0 %
$> C_{18}$: 1,6 %
unknown: 2,1 %

It is further preferred to add to the formulation the long chain fatty acids known as eicosapentaenoic acid [C20: $5\omega3$] and decosahexaenoic acid [C22:$6\omega3$]. Such a product combination is available from Roche Lipid Technology under the trade name "Ropufa '30' n-3 oil".

It has been found by microscopic studies that the formulation of the nucleic acid substance with a medium as herein described gives rise to the formation of minute, generally spherical bodies, within which, or attached to which the active ingredient is contained in a stable form and from which it is delivered at the site of action namely on or inside the cell in or from the animal body or plant or micro-organism to which it is administered.

The nucleic acid substance utilised in the method or formulation according to the present invention may comprise any one or more of the vast spectrum of nucleic acid substances as herein defined.

In a preferred form of the invention the nucleic acid substance is selected from the group comprising: DNA, RNA, DNA-RNA hybrids, oligonucleotides and synthetic nucleic acids.

The invention has not yet been demonstrated by empirical work- to be applicable to all the agents or classes of agents referred to herein. However in respect of such nucleic acid substances which have already been formulated with the aforementioned administration medium of the invention, and evaluated by different methods for the anticipated enhancement of action, no negative result has as yet been seen despite the chemical and physical diversity of the nucleic acid substances which have been investigated. The applicant thus confidently expects on the basis of these preliminary observations that the invention will find general application across the entire spectrum of the nucleic acid substances embraced by the term as herein defined and of which some examples are set out herein.

It is part of the applicant's present postulations by which it seeks to find an understanding of the invention and to which it does not wish to be bound at this stage, that while the administration medium of the present invention serves to transport the nucleic acid substance formulated therewith most efficiently through the human or animal body, that medium also plays an important role in transferring, by an as yet unexplained mechanism, the nucleic acid substance through the membranes of and into the cells thereby to cause an effective nucleic acid substance inter- and intracellular concentration of the agent rapidly to be achieved, and to be maintained. It is in this respect that the applicant believes that the present invention will find general application.

From amongst the nucleic acid substances in the form of DNA with which this invention is concerned it relates particularly to DNA fragments making up genes or parts thereof, as are used in gene therapy, and in particular

a) to supplement defective genes with 'correct' genes in order to affect the outcome of diseases, as are used in non-viral gene therapy; or
b) to transfect cells with exogenous DNA to enable expression of such exogenous DNA as proteins, as are used in biotechnology; or
c) to interfere in transcription of genomic DNA, as in therapy of diseases where over-expression of genetic material is a causative effect; or
d) to change the regulatory control of the expression of genes, as it may relate to some malignant and other diseases where regulation of gene expression is causative; or
e) to transport antisense bacterial, viral or parasitic DNA or oligonucleotides into cells, there to have an inhibitory or stimulatory action on the growth of such infectious agents or a degradative action against the genome or the mRNA of the agent.

The nucleic acid substance used according to this invention may accordingly be those coding for the expression of any desired protein, or protein classes. The invention is thus applicable to the administration of nucleic acid sequences coding for any one of the following protein classes:

chemokines
chemotactins
cytokines
enzymes
gonadotrophins
growth factors
immunoglobulins
interferons
interleukins
lipid-binding proteins
pituitary hormones
protease inhibitors
proteases
somatomedins

More particularly the nucleic acid sequence may be selected to code for any one of the following specific proteins, namely: adenosine deamidase,

bovine growth hormone (BGH)
brain-derived neurite factor (BDNF)
calcitonin

calcitonin
carboxypeptidase
catalase
cholecystokinin (CCK)
chymotrypsin
ciliary neurite transforming factor (CNTF)
clotting factor VIII
clotting factor VIII
elastase
erythropoietin (EPO)
fibroblast growth factor (acidic or arginase basic)
glucagon
glucagon-like-peptide I (GLP-1)
granulocyte colony stimulating factor .gamma.-interteron(G-CSF)
granulocyte macrophage colony stimulating factor
human growth hormone (hGH)
IL-1
IL-1 RA
IL-2
Insulin
insulin-like growth factor-1(IGF-1)
insulinotrophic hormone
insulintropin
interferon-.alpha.2B
intrinsic factor
lactase
L-asparaginase
neurite growth factor (NGF)
Ob gene product
parathyroid hormone(PTH)-like hormone
pepsin
phenylalanine ammonia lyase
platelet derived growth factor interferon-.alpha.2A (PDGF)
streptokinase
sucrase
superoxide dismutase (SOD)
thrombopoietin (TPO),
tissue necrosis factor (TNF)
tissue plasminogen activator (tPA)
transforming growth factor (TGF)
trypsin
uricase
urokinase

The disease conditions which are amenable to treatment using the present invention will be readily apparent to those skilled in the art and include those listed by German et al in the aforementioned US patent 6258789.

The method and composition of the invention may further be **characterised in that** the carrier compostion may include a peptide selective for a selected receptor on a target cell, such peptide being bound to at least one of the fatty acids or derivatives thereof in the composition. By the presence of such peptide the nanolipid vesicle with its charge of a nucleic acid substance such as DNA may be caused to be targeted to a specific site in the animal or plant body or in the cells so as to dock at at the desired locus for the release of that charge.

**PRELIMINARY HYPOTHESES OF MECHANISM OF OPERATION**

The mechanism by which nucleic acid transportation and delivery is achieved by the present invention, is currently under investigation. Some observations in this regard have been recorded above. In addition it is recorded that preliminary observations point to some additional possible explanations. The applicant again does not wish to be bound to any of the tentative explanations it may put forward at this time. It is recorded, however, that it would appear that the long chain fatty acids used in the formulation of the preparation according to the invention,or at least some of these components, form, during the manufacturing process of the medicinal formulation, very small spherical bodies, hereinafter referred to as "nanolipid vesicles". These nanolipid vesicles have dynamic characteristics in respect of the encapsulation and subsequent delivery of compounds at predicted areas in cells and organisms where the optimal utilisation of these compounds occur with resultant maximised modes of actions.

The present model for understanding the invention is that the dynamic delivery characteristics of the nanolipid vesicles are utilised efficiently to transport compounds to locations where maintenance of optimal concentrations in the organisms or cells is beneficial in combating specific genetic, acquired and infective diseases.

These beneficial effects are believed to be attributable to the dynamic characteristics of the nanolipid vesicles. The current hypothesis is that these characteristics include:

**1. The structural characteristics of the formulation of the preparation:**

Nitrous oxide and the unsaturated long chain fatty acids forming part of the administration medium are formulated by being mixed with designated nucleic acid substances or compounds to form the nanolipid vesicles containing the compound or nucleic acid substance. Two important observations have been made in this regard:

a) It was found that when the unsaturated long chain fatty acids used are 20 carbons or more, the nanolipid vesicles form spherical structures with sub-compartments similar to those seen in a sponge.

These structures are stable and it is our belief that antibodies or other ligands would fit ideally in these sub-compartments so that the nanolipid vesicles bind to specific epitopes or receptors at the target cell surface.

b) When unsaturated long chain fatty acids of 16 to 20 carbons are used, the form of the nanolipid vesicles is spherical with a dynamic field of moving autofluorescent particles surrounding the vesicles.

When nitrous oxide is omitted from the process the moving particles surrounding the nanolipid vesicles move erratically and asymmetrical movements are then detected.

It is believed that nitrous oxide is essential in stabilising the moving autofluorescent particles surrounding the nanolipid-vesicles, which is an essential characteristic to efficient compound delivery.

**2. Stability:**

The nanolipid vesicles appear to remain structurally intact after 24 months at room temperature. Any encapsulated active compounds remain encapsulated during this time. This stability feature is believed to be of substantial significance in the design of a pharmaceutical acceptable carrier of nucleic acids.

**3. Absence of cytotoxicity:**

The nanolipid vesicles have no apparent cytotoxicity. When applied to cells in culture, at applicable concentrations they appear rather to have a beneficial effect on normal cell growth.

**4. Targeting:**

It has been observed that the nanolipid vesicles with unsaturated long chain fatty acids of 16 to 20 carbons, when added to cells in culture, concentrate around the mitochondria which are in the region of the nuclear membrane. Nanolipid vesicles appear to display a natural affinity towards mictochondria. Furtermore mitochondria contain a specific transport mechanism for long chain fatty acids.

When the unsaturated fatty acids used are longer than 20 carbons, it is thought that the nanolipid vesicles target peroxisomes. It is known that peroxisomes metabolise fatty acids containing more than 20 carbons.

In *in vivo* applications, the biodisposition of nanolipid vesicle-encapsulated compounds has been found to favour the liver and spleen: Although the transport of normally readily diffusible compounds across the blood brain barrier is inhibited by the nanolipid vesicles, the nanolipid vesicles have been shown to deliver and release active compounds through the blood brain barrier to a greater extent than current commercial preparations. The nanolipid vesicles may furthermore contribute to the bioavailability of orally administered conjugations of an active compound and the nanolipid vesicles, as was shown for antibiotic compounds, which is the subject of a separate patent application. In addition, the nanolipid vesicle may contribute to the protection or stabilization of the active compound in the blood, especially in the case of nucleic acids and /or peptides being used as active compound, where the compound is prone to digestion by nucleases and proteases.

### 5. Mechanism of action:

### 5.1 Loading efficiency:

The high loading efficiency of nanolipid vesicles has been demonstrated by achieving a high degree of encapsulation of a wide range of active drugs and compounds.

Human DNA fragments have also been shown to be transported by the lipid vesicles into the nuclei of viable cells, while the nanolipid-vesicle remains intact.

### 5.2 Release:

It has been shown that the nanolipid-vesicles have very high delivery efficiencies. The high delivery efficiency relates to tissue penetration, cell absorption, internalisation of nanolipid-vesicles by cells, intra-cellular stability, and subsequent sub cellular organelle delivery.

The result of the high delivery efficiency is release of active compounds not only at membrane sites, but also at intracellular sites including the nucleus of viable cells. The result is an enhanced efficacy of the functional expression of said active compound. The nanolipid-vesicles and active compound appear to act synergistically in attaining enhanced efficacy or functional expression.

### 6. Elasticity:

Confocal laser scanning microscopy (CLSM) shows that the conformation of nanolipid-vesicles can be changed while in movement.

When the vesicles move through membranes, the conformation changes so that the intracellular nanolipid-vesicles may have other characteristics.

While moving through membranes the nanolipid-vesicles 'feed' the membranes with unsaturated long chain fatty acids which in its turn will have a positive effect on membrane bound processes. This process has a positive effect on the metabolism of the cell and thus the survival of the cells.

### 7. Dynamic inter-lipid vesicle relationships:

It has been shown that dynamic inter lipid vesicle relationships do exist. The lipid vesicles can have interchange of the compounds they carry.

They can also combine to resize themselves continuously without detriment to their stability. The inter-lipid relationship is also revealed when moving through the cellular membrane.

These interactive membrane characteristics make the movement of the vesicles through the cells optimal.

Although inter-relationships of the dynamic nano-lipid vesicles are continuously present, it has also been shown that the particles are stable in blood and body fluids for up to 5 hours.

### EXAMPLES OF THE INVENTION

Without thereby limiting the scope of the invention some examples will now be described to illustrate the invention. Two preparations which do not form part of the claimed subject matter of this application, being preparations first disclosed in the applicant's own prior patents referred to above, are first restated.

### PREPARATION 1

### Preparing an aqueous nitrous oxide solution

A pressure vessel is charged to its operating volume with water at 20°C [ambient temperature]. The vessel is connected to a supply of nitrous oxide via a flow control valve and pressure regulator. The closed vessel is supplied with nitrous oxide at a pressure of 2 bar for a period of 48 hours, it having been determined that at the aforementioned temperature the water is saturated with nitrous oxide over such period of time under the above-mentioned pressure.

A resultant solution is bottled as stock solution for use in the formulations and applications set out below.

**PREPARATION 2**

**Preparation of an aqueous nitrous oxide/Vitamin F emulsion**

30g Vitamin F ethyl ester as identified and described above was mixed with 10g Cremophor RH40, 2,2g methyl paraben, 0,08g butylated hydroxyanisole, 0,23g butylated hydroxytoluene with stirring at 80°C.
Into 942,5g of the stock nitrous oxide solution was dissolved 2,5g sodium propyl paraben and 2,5g Germall 115 [Imidurea] with stirring at room temperature.
The oily composition first described was emulsified into the aqueous solution with stirring to constitute a stock nitrous oxide/Vitamin F emulsion. It is herein referred to as the "nanolipid vesicle" formulation.

**Preparation 3**

**Preparation of a typical formulation having a non-aqueous solution of nitrous oxide in the carrier formulation.**

The manufacturing process for producing a non-aqueous formulation of a nucleic acid substance according to the invention is based on the principles and ratios of the ingredients described with reference to the manufacture of a formulation containing the dedicated nucleic acids, which can be a gene or part therof or a genome or part therof as in the case of DNA vaccines, or ODN's as in the case of the targeting of infectious agents as active ingredient
The non-aqueous nucleic acid preparation was made up based on the following protocol:

> **Step 1:** Weigh of the applicable weight of nucleic acids as defined above for the purpose required.
> **Step 2:** Weigh off and add together the Poloxyl hydrogenated castor oil to a final concentration of 11.5% (w/w) Vitamin F ethyl ester to a final concentration of 23.5% (w/w) and Polyethylene glycol 400 to a final concentration of 12% (w/w) into mixing pot 2 and heat to approximately 40°C until all the oil has melted. dl-α-Tocopherol may be added depending on the physico-chemical characteristics of the active compound, the rate of release and period of release needed. Eicosapentaenoic acid and/or decosahexaenoic acid may be added at this step.
> **Step 3**: Gas the oil mixture with nitrous oxide for 3 hours at 2 bar in the stainless steel pressure vessel in the manner described in Preparation 1 above.
> **Step 4:** Transfer the gased mixture to mixing pot 1 and heat to approximately 70°C.
> **Step 5**: Weigh off and add the Methyl paraben to a final volume of 0.5% and the Butylated hydroxytoluene 0.05% to mixing pot 1, while continuously mixing ensuring each solid is dissolved before adding the next while still maintaining the temperature at 70°C.
> **Step 6:** Remove from the heat and allow to cool down to approximately 40° C.
> **Step 7:** Add the required amount of stepwise while continuously mixing.
> **Step 8:** Gas the mixture from step 7 with nitrous oxide at 20kPa for 30 minutes with mixing until the mixture has reached room temperature.

The Nucleic acid Preparation prepared as set out above was encapsulated in soft gel capsules in the manner well known in the pharmaceutical trade as oral capsules. This preparations may be further diluted with water preferably saturated with nitrous oxide to obtain the desired ratios of nanolipid vesicle and nucleic acids.

EXAMPLE 1

STUDY TO DEMONSTRATE THE NUCLEIC ACID TRANSPORTATION AND DELIVERY CHARACTERISTICS OF THE TRANSPORT MEDIUM OF THE INVENTION

AIM:

> The scope of this study is to confirm that:

> a) nucleic acids in its various forms could be stably entrapped in the nanolipid formulation of the present invention;
> b) such entrapped nucleic acids could be transported across the cells membrane to cell nuclei, cell organelles or plasma;
> c) the nanolipid formulation medium is not cytotoxic to cells;
> d) The nanolipid formulation medium can be used to co-package various ligands with nucleic acid in the nanolipid vesicles, thereby targetting the nucleic acids to specific cell types by virtue of their cell surface antigens.

Three types of nucleic acids were selected for the investigation: Linear double stranded (ds) human DNA
Circular ds DNA:
Oligodeoxynucleotides (ODN's)

**PROTOCOL:**

Motivation for investigating linear double stranded (ds) DNA:

Using the vesicles of the nanolipid formulation medium as a delivery system for transgenes is considered to be body friendly and safe, without the cell damage caused by harsh physical methods or the disadvantages of viral vectors.

Motivation for investigating Oligodeoxynucleotids and ribozymes:

Due to the increase in drug resistance in the fight against infectious diseases, the use of antisense oligodeoxynucleotides (ODN's) for the inhibition of intracellular organism growth currently receives increasing attention. One of the ways in which ODN's can be used is to ensure the presence of ODN's of specific design in the cytoplasm. This can result in the formation of complementary base pairing between the ODN's and specific messenger RNAs, which can in turn inhibit the growth of the organisms.

Motivation for investigating circular DNA

A number of recombinant DNA vaccines use circular DNA, containing DNA engineered for vaccination against specific infectious agents. The physical state of the DNA transferred into cells by the delivery system may contribute to its survival and subsequent expression. The coiling and compaction of circular DNA differs from that of linear DNA, due to unrelieved supercoiling of the DNA. For that reason, it was considered necessary to confirm the entrapment of circular DNA into the nanolipid vesicle formulation of the invention.

**Materials and methods:**

**Nucleic Acids:**

The following nucleic acids were used in the study.

a) Human placental DNA, fragmented to sizes averaging 800bp (commercially available form Sigma);
b) recombinant circular DNA from vaccinia virus, obtained from University of Cape Town;
c) oligodeoxynucleotide designed by the Dept of Biochemistry of the University of Pretoria, South Africa. The ODN's were commercially manufactured with the inclusion of a FITC fluorescent label inherent in the design.

**Cells:**

The oncogenic cell line used in the study, designated UCT-1, was specifically cultured the University of Cape Town. Primary UCT-1 cells were cultured form a metastases of a patient's melanoma. The human melanoma cultured cells has all the major characteristics of fast growing cancer cells. The cells grow in adherent fashion.
The dendritic cells used were either the cell line THP-1 (ATCC) or primary macrophages, isolated and cultured from the blood of a donor.
The ODN approach was tested for the malaria parasite Plasmodium Falciparum. The mixture was fed to Plasmodium infected human red blood cell cultures, cultured in the presence of human serum from a donor.

**Reagants:**

Reagents used were obtained from commercial vendors, as specified in the procedure below. Fluorophores were from Molecular Probes (Holland) and included nile red, acridine orange, TMRM, ethidium bromide, Alexa Green, Baclight, carboxy fluorescien and $DIOC_6$.

**Analysis**

Confocal laser scanning microscopy (CLSM) was used to observe entrapment of nucleic acids in the nanolipid formulation medium. The dual wavelength CLSM was done on a Nikon PCM2000 microscope, with Kr/Ar and He/Ne lasers or a Zeiss LSM410, using the same lasers. Pinhole size generally used as 5$\mu$m and smaller. Objectives used were oil-dispersion 60x and 100x. Light and fluorescent microscopy on a Nikon TE300 inverted microscope was digitally captured, using Nikon DMX video and Nikon Coolpix900 digital cameras.

To measure the inhibition of parasite growth when using antisense ODN's, the determination of % parasitaemia was by counting of the number of intracellular parasites per number of erythrocytes and by FACS analysis.

**Typical preparation of DNA for encapsulation in nanolipid vesicle transport medium:**

1. Six $\mu$g (10$\mu$l) of isolated circular vaccinia virus DNA was labelled fluorescently with 1 $\mu$l of a 0.1ug/ml Ethidium Bromide solution. Ethidium Bromide interchelates with double stranded DNA, resulting in green fluorescence at the laser excitation wavelength used.

Ten $\mu$g of fragmented double stranded human placental DNA, fragmented to sizes averaging 800bp, was also labelled as above.

One $\mu$g of FITC-labelled oligodeoxinudeotides were used. In this case, the ODN's were not labelled with ethidium bromide.

2. Free fluorophores were removed by washing procedures, except where different methods are described. The typical washing procedure used is described below:

Free ethidium bromide was removed by ethanol precipitation of the DNA in the following manner:

a) Three volumes of 99% ethanol and sodium acetate to a final concentration of 0.2M were typically added to the labelled DNA. The mix was left at overnight at -20°C or 30 minutes at -80°C, after which the DNA was collected by micro-centrifugation for 30 minutes at 15 000 rpm.

b) The collected nucleic acids were washed twice with 70% ethanol and air dried in laminar flow cabinet.

c) The DNA was resuspended in 10ul of sterile water and checked by CLSM to confirm that the fluorescent labelling was successful.

**Using the nanolipid vesicle formulation as a medium to encapsulate the labelled DNA.**

1. Nanolipid vesicle formulation was filter steriized, using 0.45um filters.

2. Typically between 100 and 500 $\mu$l pf the nanolipid vesicle formulation was then mixed with the fluorophore Nile red (Molecular Probes, Holland) to a final concentration ranging from 10nM to 50nM. The amount of nanolipid vesicle "formulation and Nile red used depended on the composition of the formulation as well as the proposed cell type used. In the nanolipid vesicle formulation Nile red-associated fatty acids fluoresce in the red spectrum when excited by laser energy. Nile red not associated with or bound to fatty acids, and other lipids do not emit red fluorescence under those conditions. DNA itself fluoresces in the green spectrum.

3. The presence of fluorescent transport vesicles in the nanolipid transport medium was confirmed by CLSM.

4. Typically, the resuspended green fluorescent DNA was entrapped into nanolipid formulation medium by thorough mixing with between 100- 500 $\mu$l of Nile red abetted nanolipid vesicle formulation medium, depending on the DNA load needed. Mixing was by 30 minutes sonication in a sonicating bath with low frequency, or by vigorous vortexing, or by slow homogenization. The mixing temperatures can be varied, but was typically between room temperature and 40°C.

5. Between 5 and 10$\mu$l of the Nanolipid formulation medium with the entrapped DNA was spread thinly on a very thin glass cover slip (see below for specifications).

6. Encapsulation of the various forms of DNA referred to above into the vesicles of the nanolipid transport medium was confirmed by CLSM. Confirmation of the sizes of the entrapped DNA was done by horizontal agarose gel electrophoresis.

The nanolipid transport medium was concentrated by 34% for use with the recombinant Vaccinia DNA and 50$\mu$l of the concentrated medium was pre-labelled with nile red.

This part of the study accordingly confirms that nucleic acid substances in the form of the various forms of DNA are properly associated with the nanolipid vesicles of the formulation medium.

**Delivery of Exogenous DNA into the nucleus of cells:**

One of the major factors in determining the level of expression of the genes transferred into target cells are the survival and delivery of exogenous DNA to the nucleus. The procedure described below for the UCT-1 melanoma cells is an example and can with some modifications directly be applied the transfer of other types of nucleic acid to other cell types, such as dendritic cells.

**Preparation of Melanoma cells for DNA transfer:**

UCT-1 cells, stored in liquid Nitrogen in cryovials in the presence of 90% foetal bovine serum and 10% DMSO, were cultured in the following manner:

1. Working as quickly as possible, the cryogenic vial is transferred to a 37°C water bath.
2. Using a sterile 10 ml polypropylene pipette and Pipettes®-akku the content of the cryovial is transferred to centrifuge tube suitable for tissue cultures.
3. The cells were diluted with 2 ml 37°C media comprising of RPMI + 10% FBS (foetal bovine serum) and 0,05 % Gentamicin Sulphate.
4. The cells and media were gently mixed by shaking and then centrifuged for 1 min to loosely precipitate the cells.
5. The media was removed to eliminate any DMSO and replaced with 2 ml 37°C media comprising of RPMI + 10% FBS and 0,05 % Gentamicin Sulphate.
6. Cells were gently resuspended, using a wide mouth pipette.
7. The cells were plated in sterile polystyrene (60 x 15mm) tissue culture petri dishes.
8. Cells were cultured in Shellab $CO_2$ incubator at 37°C, 5% $CO_2$, and a relative humidity (RH) of $\pm$ 89%.

When the cells reached confluence, they were typically plated onto 31x 0.017mm glass cover slips with a refractive index suitable for Confocal Laser Scanning Microscopy (CLSM) in the following manner:

9. A petri dish with a confluent layer of cells was removed from the incubator and in a flow cabinet, the media was removed under vacuum.
10. Two ml Trypsin /EDTA (Gibco/BRL ) was added to the dish and the cells returned for incubation in order to reverse adherence of the cells to the petri dish. Adherence of the cells to the petri dish was checked by light microscopy every two minutes.
11. During this time, cover slips with the specifications as described above, were sterilized. Sterilizing was done by dipping the cover slips in 70 % ethanol and flaming the cover slip after the alcohol evaporated. Six sterilized cover slips were placed in a polypropylene 6 well multi-plate.
12. When the cells have lost adherence, 5ml media (RPMI + 10% FBS and 0,05 % Gentamicin Sulphate) at 37°C were added to the petri dish to dilute the Trypsin/EDTA.
13. The petri dish content was transferred to a centrifuge tube, and the cells were collected by centrifugation at 3000 r.p.m. for 1 min. The supernatant was removed under vacuum.
14. Three ml media (RPMI + 10% FBS and 0,05 % Gentamicin Sulphate) was carefully added and the cells gently resuspended.
15. In each well, onto the glass coverslip, 0.5 ml of the cell suspension was plated.
16. Adherence of the cells was checked by microscopy and as soon as the cells were adherent, media was added to a final volume of 1 ml.
17. The cells were incubated overnight at 37°C, 5% $CO_2$, and a RH of $\pm$ 89%.
18. If the cells cultured on the cover slip were determined to be typical of its parent cells by microscopy, the procedure continued as follows:
19. One of the cover slips containing the adherent cells were placed into a cellular flow cell with the side with the cells facing up. The stainless steel flow cell was specifically designed to fit the stage of the Nikon PCM2000 confocal laser scanning microscope. The cover slip with its adherent cells then forms the thin glass base of the flow cell. The leak-free flow cell is completed with the use of sterilized rubber O-rings, a polymer spacer and a second sterile cover slip. Working very quickly, 1 ml of the described media at 37°C was very gently added to the cells in the flow cell. The flow cell was checked for any tiny cracks in the cover slips and then fixed onto the stage of the CLSM and the cells left to stabilize.
20. To determine cytotoxicity of the nanolipid formulation medium, the same procedure was followed, but with the omission of any nucleic acids. A range of concentrations of the nanolipid vesicle transport medium was investigated, form undiluted to a 1000 fold dilution. The cells were exposed to the transport medium for periods ranging from 2 days to 14 days, with concomitant passaging of the cells when necessary.

Note: Dendritic cell, in this case THP-1 macrophages, were cultured and treated in a similar fashion, with the exception that the Gentamycin was omitted or used at a much reduced concentration, and only when necessary. When primary human macrophages were used, the foetal bovine serum was replaced with 20% AB human serum.

**Transfer of Vaccinia Virus DNA into melanoma cell nuclei by the Nanolipid formulation medium.**

Note that this procedure can with some modifications be applied to double stranded linear DNA for delivery into the nuclei or cell organelles or the cytoplasm of other cell types as well.

1. Labelled DNA-loaded nanolipid vesicles were isolated by differential micro-centrifugation. Care was taken that the internal water phase of the vesicles was not depleted. During the differential centrifugation steps, the status of the internal water phase was monitored by CLSM.
2. Typically between 5 and 100 ul of the labelled DNA-loaded MZL vesicles were carefully and gradually added to the medium of the melanoma cells by the inlet in the prepared flow cell or by removing the top glass cover slip of the flow cell.
3. Transfer of the DNA in the nanolipid vesicle formulation were allowed to continue for between 15 to 60 minutes, after which time the cell medium was removed and replaced with fresh cell medium that contained no DNA-loaded nanolipid formulation medium.
4. Progress of DNA transport into the melanoma cell nuclei was followed and confirmed by CLSM.

This part of the investigation thius confirmed that the DNA associated nanolipid vesicles duly entered the melenoma cell.
Furthermore, the release of the labelled DNA from the nanolipid vesicle transport medium inside the cell nuclei and inside the cell cytoplasm were also confirmed by CLSM.

**Attachment of a peptide to the Nanolipid formulation medium Vesicles.**

1. The procedure described below can be used for a variety of macromolecules, including antibodies, enzymes and glycoproteins.
2. 100 mg of the peptide was pre-labelled with Fluorescein Diacetate (FA) to a final concentration of 10nM. FA emits green fluorescence when exposed to the applicable laser line.
3. The peptide was entrapped in the concentrated Nanolipid formulation medium as described above.
4. The incorporation of the peptide into Nanolipid formulation medium vesicles and sponges were followed by light video microscopy and CLSM as described above.

**Results:**

**1. Entrapment of Nucleic Acids by the Nanolipid formulation medium**

The entrapment of nucleic acids in the nanolipid transport medium vesicles was illustrated by CLSM. The nucleic acids were entrapped in the vesicles. Nucleic acids in the process of being entrapped were visualized by its green fluorescence. Co-localization between the Nanolipid formulation medium vesicles and green nucleic acids were similarly visualized. The loading efficiencies were high - between 85% and 95% at the concentrations of nucleic acid and Nanolipid formulation medium used. Nearly all vesicles showed entrapped DNA.
The percentage loading efficiency can be calculated by

$$\frac{\text{Nr of vesicles containing nucleic acid}}{\text{Total nr of vesicles}} \times 100$$

Most nucleic acids are entrapped in the hydrophilic centre of the vesicles, sponges or depots. Circular DNA can be visualized where the DNA follows the contour of individual vesicles, sightly associated with the interior layer of the vesicles membrane.

**Stability of nucleic acids entrapped in Nanolipid formulation medium**

The stability of nucleic acids in the Nanolipid formulation medium was confirmed by CLSM investigation: at room temperature, there was no obvious degradation or digestion of the entrapped nucleic acid for several weeks after preparation of the entrapped nucleic acids. The delivery of the nucleic acids to cells were typically done 1 week after entrapment of the nucleic acids in the nanolipid vesicle transport medium.

**Delivery of Exogenous DNA into the nucleus of cells:**

In vitro experiments with both dendritic and oncogenic cell lines confirmed the uptake of exogenous nucleic acids entrapped in the nanolipid vesicle transport medium. In both the oncogenic Melanoma UCT-1 cells, and in the dendritic THP-1 cells, delivery to the cytoplasm, the mitochondrial genome and the nucleus could be demonstrated.

In the THP-1 macrophage cell line, the uptake of the entrapped exogenous nucleic acid was by two mechanisms: a process of phagocytosis, or by transport across the cell membrane. In the melanoma cell, transport to the nucleus and mitochondria were across the cell membrane, by way of the cytoplasm. The presence of a few intact nanolipid vesicles in the cytoplasm could be demonstrated.

Release of the nucleic acids from the MZL vesicles was demonstrated in both the cytoplasm and the nucleus by optical sectioning and 3D-reconstruction of the cells on the CLSM. The release of the nucleic acid from the vesicles in the nucleus is necessary before DNA can be integrated into the endogenous genome for transcription. Release in the cytoplasm is especially important in the case of the ODN's, where the ODN's would result in inhibition of protein synthesis of infective agents. Research with both susceptible and drug-resistant strain of the intracellular parasite Plasmodium Falciparum in the in vitro human erythrocyte system showed that antisense oligodeoxynucleotides, entrapped by the nanolipid vesicle transport medium, can be used to inhibit the growth of the parasites in the red blood cell cultures.

**Targeting of the Nanolipid formulation medium Vesicles:**

The attachment of peptides into nanolipid formulation vesicles was demonstrated. The current data suggests that the peptides are attached to outer membrane of the vesicles by strong electrotatic bonds. The attachment to the outer vesicle membrane has the advantage that recognition between the peptide and the cell recognition antigen / receptor can occur without any steric hindrance. The peptide used in the experiments described above, was targeted to melanoma cells and fibroblasts. In additional experiment, using another ligand, the ligand have been shown to retain receptor recognition activity whilst packaged in nanolipid vesicle transport medium. These packaged vesicles can thus be targeted to cells containing the appropriate receptor. It is our belief that the same will hold true for other macromolecules, such as antibodies.

**Toxicity and Cytotoxicity:**

**Cytotoxicity**: No cytoxicity was observed at the concentrations used under experimental conditions (1:300 to 1:1000 dilution of the Nanolipid formulation medium, diluted with RPMI medium). No adverse effects were observed at these concentrations. The cells were sensitive to undiluted Transport Medium if exposed to it for long periods (>1 day).

**General safety:**

VITAMIN F: Since the constituents in vitamin F comprise the n-essential fatty acids, which are fundamental to normal physiological processes, it is unlikely to be inherently toxic. It could theoretically be toxic if consumed at levels far higher than those normally available to the body. For example, the estimated endogenous rate of formation of the intermediate, gamma linolenic acid within the body of a normal adult was put at 100 - 1000 mg, or around 2-20mg/day. It was also estimated that daily intake of GLA in a human fully breast fed infant is of the order of 20-80mg/kg/day. Based on these estimates, it seems unlikely that doses of less than 100mg/kg/day will have any toxicity (Horrobin DF. Nutritional and medical importance of gamma-linolenic acid. Lipid Res, 1992. 31(2): 163-194). Long term animal toxicity studies have shown that doses of evening primrose oil of up to 5 ml or 10ml/kg/day given for up to two years has no toxicity (Everett DJ, Perry CJ, Bayliss P. Med Sci Res.1988; 16:865-866).

Cremophor: From the available literature' it is concluded that Cremophor administered intravenously may elicit certain dose-dependent toxicity characteristics. There is no evidence to suggest that the same is true for oral administration forms. In fact, in a study using oral administration of the drug paclitaxel in combination with Cremaphor EL, results showed undetectable plasma Cremaphor levels. It was concluded that this finding would have a beneficial influence on the safety of treatment with the MZL Nucleic Acid Transport Medium, especially when it is used in its prodrug form for oral administration. In this form, any Exipient (see Textbook of Exipients) may be added, which in itself refers to the safety and indicates the prescribed dosages.

**Conclusions**

The nanolipid vesicle administration or transport medium is a formulation capable of encapsulating macromolecules and delivering them with high efficacy to target sites. Furthermore, the encapsulating vesicles have been shown to penetrate through the cell membrane and to migrate to cell organelles. This study shows the efficient transport and delivery of DNA to such cell organelle, including the nuclei of the cells.

The major steps in the transfection process, ie the entry of DNA into the cell, the trafficking of the transgene through the cytoplasm, including both endosomal and lysosomal compartments, the entry of DNA into the nucleus or cell organelle, such as the mitochondria or the targeting of the nucleic acid to an intracellular infective agent, such as parasite or the targeting to the nucleic acids of such infective agents in the cellular cytoplasm and the ability to escape intracellular mechanisms designed to purge foreign nucleic acids, has in one way or another been addressed by these studies, with positive results.

This product has several unique advantages:

(i) It is a highly effective gene transfer vector and much more efficient than conventional products currently on the market

(ii) One of the most interesting and exciting properties is that it can be used to package ligands so that vesicles can be targeted to specific cell surface receptors for uptake by these cells.

(iii) It can be used to transfer molecules trans-dermally, orally or through the any of the mucous membranes without the need for sophisticated procedures.

**Claims**

1. A method for the administration of a nucleic acid substance to the cells of a plant or a micro-organism or for the in vitro administration of a nucleic acid substance to the cells of an animal, the method being **characterised in that** the nucleic acid substance is formulated with an administration medium which comprises a solution of nitrous oxide gas in a physiologically acceptable carrier solvent for the gas and which administration medium includes at least one fatty acid or ester or other suitable derivative thereof selected from the group consisting of oleic acid, linoleic acid, alpha-linolenic acid, gamma-linolenic acid, arachidonic acid, eicosapentaenoic acid [C20: 5$\omega$3], decosahexaenoic acid [C22: 6$\omega$3], ricinoleic acid and derivatives thereof selected from the group consisting of the C1 to C6 alkyl esters thereof, the glycerol-polyethylene glycol esters thereof and the reaction product of hydrogenated natural oils composed largely of ricinoleic acid based oils, such as castor oil, with ethylene oxide.

2. A preparation for use in introducing a nucleic acid substance into the cells of an animal, a plant or a micro-organism **characterised in that** the preparation comprises a formulation of the nucleic acid in an administration medium which comprises a solution of nitrous oxide gas in a physiologically acceptable carrier solvent for the gas and which administration medium includes at least one fatty acid or ester or other suitable derivative thereof selected from the group consisting of oleic acid, linoleic acid, alpha-linolenic acid, gamma-linolenic acid, arachidonic acid, eicosapentaenoic acid [C20: 5$\omega$3], decosahexaenoic acid [C22: 6$\omega$3], ricinoleic acid and derivatives thereof selected from the group consisting of the C1 to C6 alkyl esters thereof, the glycerol-polyethylene glycol esters thereof and the reaction product of hydrogenated natural oils composed largely of ricinoleic acid based oils, such as castor oil with ethylene oxide.

3. The method of claim 1 or the preparation of claim 2 wherein the essential fatty acid or ester thereof, component of the composition comprises a mixture of esters of the fatty acids listed above and is prefereably constituted by the complex known as Vitamin F Ethyl Ester having a typical fatty acid distribution as follows:

$< C_{16}$: 0
$C_{16.0}$ : 8,3 %

$C_{18.0}$ : 3,5 %
$C_{18.1}$ : 21,7 %
$C_{18.1}$ : 34,8 %
$C_{18.4}$ : 28,0 %
$> C_{18}$: 1,6 %.
unknown: 2,1 %.

4.   The method or the preparation of claim 3 wherein the administration medium further includes eicosapentaenoic acid [C20: 5ω3] and/or decosahexaenoic acid [C22: 6ω3] as additional long chain fatty acids.

5.   The method of claim 1 or the preparation of claim 2 wherein the carrier solvent is water (preferably deionised water) or any of the pharmaceutically acceptable alcohols, ethers, polymers such as a polyethyleneglycol or the like or an oil which which is preferably an organic oil which organic oil is further preferably an essential oil based on long chain fatty acids having between 14 and 22 carbon atoms in the fatty acid and is preferably of natural origin and most preferably a plant oil rich in gamma linolenic acid [GLA].

6.   The method of claim 1 or the preparation of claim 2 wherein the nucleic acid substance is formulated in a liquid presentation and wherein the formulation incorporates, as part of the administration medium, water or acceptable other liquid solvent into which the nitrous oxide is dissolved, preferably to saturation, and wherein the fatty acid or ester thereof is dissolved or suspended or emulsified along with the nucleic acid substance.

7.   The method of claim 1 or formulation of claim 2 wherein the carrier solvent for the nitrous oxide gas is essentially non-aqueous and composed of the least one fatty acid or ester thereof selected from the group consisting of oleic acid, linoleic acid, alpha-linolenic acid, gamma-linolenic acid, arachidonic acid, eicosapentaenoic acid [C20: 5ω3], decosahexaenoic acid [C22: 6ω3], ricinoleic acid and derivatives thereof selected from the group consisting of the C1 to C6 alkyl esters thereof, the glycerol-polyethylene glycol esters thereof and the reaction product of hydrogenated natural oils composed largely of ricinoleic acid based oils with ethylene oxide, required to be part of the formulation.

8.   The formulation of claim 7 wherein the nucleic acid substance is formulated to be suitable to be applied as a topical, buccal or vaginal cream or ointment, or skin patch or as an intravenous, intramuscular or subcutaneous injection, or as a suppository, or to be administered as an oral, nasal, or pulmonary preparation.

9.   The preparation of claim 2 wherein the nucleic acid substance is formulated to be applied as a topical, buccal or vaginal cream or ointment, or skin patch or as an intravenous, intramuscular or subcutaneous injection, or as a suppository, or to be administered as an oral, nasal, or pulmonary preparation and wherein the formulation used in making up such cream, ointment, injectable formulation, suppository, or oral, nasal, or pulmonary preparation, incorporates, along with the nucleic acid substance to be enhanced, a quantity of water containing, and preferably saturated with, nitrous oxide, the long chain fatty acid or ester thereof and the nucleic acid substance formulated therewith, and, optionally further incorporates such additional excipients and carriers as are conventionally used in the pharmaceutical trade in making up such dosage forms.

10.   The method of claim 1 or the preparation of claim 2 wherein the nucleic acid substance is selected from the group consisting of DNA, RNA, DNA-RNA hybrids, oligonucleotides and synthetic nucleic acids, and is preferably a DNA sequence coding for the expression of any one of the following protein classes: chemokines, chemotactins, cytokines, enzymes, gonadotrophins, growth factors, immunoglobulins, interferons, interleukins, lipid-binding proteins, pituitary hormones, protease inhibitors, proteases, and somatomedins.

11.   The method of claim 1 or the composition of claim 2 further **characterised in that** the composition includes a peptide antibody or other peptide derivative selective for a selected receptor or antigen or protein binding element on a target cell, such peptide being bound to at least one of the fatty acids or derivatives thereof in the composition.

**Patentansprüche**

1.   Verfahren zum Verabreichen einer Nucleinsäuresubstanz an die Zellen einer Pflanze oder eines Mikroorganismus oder zur In-vitro-Verabreichung einer Nucleinsäuresubstanz an die Zellen eines Tieres, wobei das Verfahren **dadurch gekennzeichnet ist, dass** die Nucleinsäuresubstanz mit einem Verabreichungsmedium formuliert ist, das eine Lösung von Distickstoffoxidgas in einem physiologisch akzeptablen Trägerlösungsmittel für das Gas umfasst, wobei das Verabreichungsmedium wenigstens eine Fettsäure oder ein Ester oder ein anderes geeignetes Derivat davon beinhaltet, ausgewählt aus der Gruppe bestehend aus Ölsäure, Linolsäure, Alphalinolensäure, Gammalinolensäure, Arachidonsäure, Eicosapentaensäure [C20: 5ω3], Decosahexaensäure [C22:6ω3], Rizinolsäure und Derivaten davon, die ausgewählt sind aus der Gruppe bestehend aus den C1- bis C6-Alkylestern davon, den Glycerol-Polyethylen-Glykolestern davon und dem Reaktionsprodukt von hydrierten natürlichen Ölen, die größtenteils aus Ölen auf der Basis von Rizinolsäure bestehen, wie Rizinusöl, mit Ethylenoxid.

2. Präparat zur Verwendung bei der Einleitung einer Nucleinsäuresubstanz in die Zellen eines Tieres, einer Pflanze oder eines Mikroorganismus, **dadurch gekennzeichnet, dass** das Präparat eine Formulierung der Nucleinsäure in einem Verabreichungsmedium umfasst, das eine Lösung von Distickstoffoxidgas in einem physiologisch akzeptablen Trägerlösungsmittel für das Gas umfasst, wobei das Verabreichungsmedium wenigstens eine Fettsäure oder ein Ester oder ein anderes geeignetes Derivat davon beinhaltet, ausgewählt aus der Gruppe bestehend aus Ölsäure, Linolsäure, Alphalinolensäure, Gammalinolensäure, Arachidonsäure, Eicosapentaensäure [C20: 5ω3], Decosahexaensäure [C22:6ω3], Rizinolsäure und Derivaten davon, die ausgewählt sind aus der Gruppe bestehend aus den C1- bis C6-Alkylestern davon, den Glycerol-Polyethylen-Glykolestern davon und dem Reaktionsprodukt von hydrierten natürlichen Ölen, die größtenteils aus Ölen auf der Basis von Rizinolsäure bestehen, wie Rizinusöl, mit Ethylenoxid.

3. Verfahren nach Anspruch 1 oder Präparat nach Anspruch 2, wobei die Komponente 'essentielle Fettsäure oder ein Ester davon' der Zusammensetzung ein Gemisch aus Estern der oben aufgeführten Fettsäuren umfasst und vorzugsweise aus dem als Vitamin-F-Ethylester bekannten Komplex mit der folgenden typischen Fettsäureverteilung besteht:

$< c_{16}$: 0
$C_{16,0}$: 8,3 %
$C_{18,0}$: 3,5 %
$C_{18,1}$: 21,7 %
$C_{18,2}$: 34,8 %
$C_{18,4}$: 28,0 %
$> C_{18}$: 1,6 %
unbekannt: 2,1 %

4. Verfahren oder Präparat nach Anspruch 3, wobei das Verabreichungsmedium ferner Eicosapentaensäure [C20: 5ω3] und/oder Decosahexaensäure [C22: 6ω3] als zusätzliche langkettige Fettsäuren enthält.

5. Verfahren nach Anspruch 1 oder Präparat nach Anspruch 2, wobei das Trägerlösungsmittel Wasser (vorzugsweise deionisiertes Wasser) oder ein beliebiger/s der pharmazeutisch akzeptablen Alkohole, Ether, Polymere wie Polyethylenglykol oder dergleichen oder ein Öl ist, das vorzugsweise ein organisches Öl ist, wobei das organische Öl ferner vorzugsweise ein ätherisches Öl auf der Basis langkettiger Fettsäuren mit 14 bis 22 Kohlenstoffatomen in der Fettsäure und vorzugsweise natürlichen Ursprungs und am bevorzugtesten ein an Gammalinolensäure [GLA] reiches Pflanzenöl ist.

6. Verfahren nach Anspruch 1 oder Präparat nach Anspruch 2, wobei die Nucleinsäuresubstanz in einer flüssigen Darreichung formuliert ist und wobei die Formulierung als Teil des Verabreichungsmediums Wasser oder ein anderes akzeptables flüssiges Lösungsmittel enthält, in dem das Distickstoffoxid gelöst wird, vorzugsweise bis zur Sättigung, und wobei die Fettsäure oder ein Ester davon zusammen mit der Nucleinsäuresubstanz gelöst oder suspendiert oder emulgiert wird.

7. Verfahren nach Anspruch 1 oder Formulierung nach Anspruch 2, wobei das Trägerlösungsmittel für das Distickstoffoxidgas im Wesentlichen nicht wässrig ist und aus der wenigstens einen Fettsäure oder einem Ester davon besteht, ausgewählt aus der Gruppe bestehend aus Ölsäure, Linolsäure, Alphalinolensäure, Gammalinolensäure, Arachidonsäure, Eicosapentaensäure [C20: 5ω3], Decosahexaensäure [C22:6ω3], Rizinolsäure und Derivaten davon, die ausgewählt sind aus der Gruppe bestehend aus den C1- bis C6-Alkylestern davon, den Glycerol-Polyethylen-Glykolestern davon und dem Reaktionsprodukt von hydrierten natürlichen Ölen, die größtenteils aus Ölen auf der Basis von Rizinolsäure bestehen, mit Ethylenoxid, die Bestandteil der Formulierung sein müssen.

8. Formulierung nach Anspruch 7, wobei die Nucleinsäuresubstanz so formuliert ist, dass sie zur Verwendung als topische, bukkale oder vaginale Creme oder Salbe oder als Hautpflaster oder als eine intravenöse, intramuskuläre oder subkutane Injektion oder als ein Suppositorium geeignet ist oder als orales, nasales oder pulmonales Präparat verabreicht werden kann.

9. Präparat nach Anspruch 2, wobei die Nucleinsäuresubstanz so formuliert ist, dass sie als topische, bukkale oder vaginale Creme oder Salbe oder als Hautpflaster oder als intravenöse, intramuskuläre oder subkutane Injektion oder als Suppositorium verwendet oder als orales, nasales oder pulmonales Präparat verabreicht werden kann, wobei die zur Herstellung einer/s solchen Creme, Salbe, injizierbaren Formulierung, Suppositoriums oder oralen, nasalen oder pulmonalen Präparats verwendete Formulierung zusammen mit der zu verstärkenden Nucleinsäuresubstanz eine Wassermenge, die Distickstoffoxid, die langkettige Fettsäure oder ein Ester davon und die damit formulierte Nucleinsäuresubstanz enthält und vorzugsweise damit gesättigt ist, und optional ferner zusätzliche Exzipienten und Träger einschließt, die üblicherweise in der Pharmazie zur Herstellung solcher Dosierungsformen verwendet werden.

10. Verfahren nach Anspruch 1 oder Präparat nach Anspruch 2, wobei die Nucleinsäuresubstanz ausgewählt wird aus der Gruppe bestehend aus DNA, RNA, DNA-RNA-Hybriden, Oligonucleotiden und synthetischen Nucleinsäuren und vorzugsweise eine DNA-Sequenz ist, die die Expression von einer der folgenden Proteinklassen kodiert: Chemokine, Chemotaktine, Zytokine, Enzyme, Gonadotropine, Wachstumsfaktoren, Immunglobuline, Interferone, Interleukine, Lipidbindungsproteine, Hypophysenhormone, Proteaseinhibitoren, Proteasen und Somatomedine.

11. Verfahren nach Anspruch 1 oder Zusammensetzung nach Anspruch 2, das/die ferner **dadurch gekennzeichnet ist, dass** die Zusammensetzung einen Peptidantikörper oder ein anderes Peptidderivat beinhaltet, der/das im Hinblick auf ein selektiertes Rezeptor- oder Antigen- oder Proteinbindeelement auf einer Targetzelle selektiv ist, wobei ein solches Peptid an wenigstens eine der Fettsäuren oder Derivate davon in der Zusammensetzung gebunden ist.

## Revendications

1. Procédé d'administration d'une substance d'acide nucléique aux cellules d'une plante ou d'un micro-organisme ou pour l'administration in-vitro d'une substance d'acide nucléique aux cellules d'un animal, le procédé étant **caractérisé en ce que** la substance d'acide nucléique est formulée avec un milieu d'administration qui comprend une solution de gaz d'oxyde nitreux dans un solvant support physiologiquement acceptable pour le gaz et lequel milieu d'administration comporte au moins un acide gras ou un ester ou autre dérivé convenable de celui-ci sélectionné dans le groupe consistant en acide oléique, acide linoléique, acide alpha-linolénique, acide gamma-linolénique, acide arachidonique, acide eicosapentaénoïque [C20: 5ω3], acide décosahexaénoïque [C22: 6ω3], acide ricinoléique et des dérivés de ceux-ci sélectionnés dans le groupe consistant en les esters alkyles C1 à C6 de ceux-ci, les esters glycérol-polyéthylène glycol de ceux-ci et le produit de réaction d'huiles naturelles hydrogénées composées largement d'huiles à base d'acide ricinoléique, telles que l'huile de ricin, avec l'oxyde d'éthylène.

2. Préparation destinée à être utilisée pour l'introduction d'une substance d'acide nucléique dans les cellules d'un animal, d'une plante ou d'un micro-organisme, la préparation comprenant une formulation de l'acide nucléique dans un milieu d'administration qui comprend une solution de gaz d'oxyde nitreux dans un solvant support physiologiquement acceptable pour le gaz et lequel milieu d'administration comporte au moins un acide gras ou un ester ou autre dérivé convenable de celui-ci sélectionné dans le groupe consistant en acide oléique, acide linoléique, acide alpha-linolénique, acide gamma-linolénique, acide arachidonique, acide eicosapentaénoïque [C20: 5ω3], acide décosahexaénoïque [C22: 6ω3], acide ricinoléique et des dérivés de ceux-ci sélectionnés dans le groupe consistant en les esters alkyles C1 à C6 de ceux-ci, les esters glycérol-polyéthylène glycol de ceux-ci et le produit de réaction d'huiles naturelles hydrogénées composées largement d'huiles à base d'acide ricinoléique, telles que l'huile de ricin, avec l'oxyde d'éthylène.

3. Procédé selon la revendication 1 ou préparation selon la revendication 2, dans lequel le composant d'acide gras essentiel ou son ester de la composition comprend un mélange d'esters des acides gras énumérés ci-dessus et est de préférence constitué par le complexe appelé Vitamine F Ester Ethylique, ayant la distribution d'acides gras caractéristique suivante :

$<C_{16}$ : 0
$C_{16.0}$ : 8,3%
$C_{18.0}$ : 3,5%
$C_{18.1}$ : 21,7%

C$_{18.2}$ : 34,8 %
C$_{18.4}$ : 28.0 %
> C$_{18}$ : 1,6%
inconnu : 2,1 %

4. Procédé ou préparation selon la revendication 3, dans lequel le milieu d'administration comporte en outre de l'acide eicosapentaénoïque [C20: 5ω3] et/ou de l'acide décosahexaénoïque [C22: 6ω3] sous forme d'acides gras à longue chaîne supplémentaires.

5. Procédé selon la revendication 1 ou préparation selon la revendication 2, dans lequel le solvant support est de l'eau (de préférence de l'eau déminéralisée) ou n'importe lequel des alcools, éthers, polymères pharmaceutiquement acceptables, tels que le polyéthylèneglycol ou assimilé ou une huile qui est de préférence une huile organique, laquelle huile organique étant de préférence une huile essentielle basée sur des acides gras à longue chaîne ayant entre 14 et 22 atomes de carbone dans l'acide gras et étant de préférence d'origine naturelle et idéalement une huile végétale riche en acide gamma-linolénique [GLA].

6. Procédé selon la revendication 1 ou préparation selon la revendication 2, dans lequel la substance d'acide nucléique est formulée dans une présentation liquide et dans lequel la formulation incorpore, comme élément du milieu d'administration, de l'eau ou n autre solvant liquide acceptable dans lequel l'oxyde nitreux est dissous, de préférence jusqu'à saturation, et dans lequel l'acide gras ou ester de celui-ci est dissous ou en suspension ou émulsifié avec la substance d'acide nucléique.

7. Procédé selon la revendication 1 ou formulation selon la revendication 2, dans lequel le solvant support du gaz d'oxyde nitreux est essentiellement non aqueux et composé de l'au moins un acide gras ou son ester sélectionné dans le groupe consistant en acide oléique, acide linoléique, acide alpha-linolénique, acide gamma-linolénique, acide arachidonique, acide eicosapentaénoïque [C20: 5ω3], acide décosahexaénoïque [C22: 6ω3], acide ricinoléique et des dérivés de ceux-ci sélectionnés dans le groupe consistant en les esters alkyles C1 à C6 de ceux-ci, les esters glycérol-polyéthylène glycol de ceux-ci et le produit de réaction d'huiles naturelles hydrogénées composées largement d'huiles à base d'acide ricinoléique, telles que l'huile de ricin, avec l'oxyde d'éthylène, devant faire part de la formulation.

8. Formulation selon la revendication 7, la substance d'acide nucléique étant formulée pour pouvoir être appliquée comme crème ou onguent topique, buccal ou vaginal, ou timbre transdermique ou injection intraveineuse, intramusculaire ou sous-cutanée, ou comme suppositoire, ou pour être administrée sous forme de préparation orale, nasale ou pulmonaire.

9. Préparation selon la revendication 2, dans laquelle la substance d'acide nucléique est formulée pour pouvoir être appliquée comme crème ou onguent topique, buccal ou vaginal, ou timbre transdermique ou injection intraveineuse, intramusculaire ou sous-cutanée, ou comme suppositoire, ou pour être administrée sous forme de préparation orale, nasale ou pulmonaire et dans laquelle la formulation utilisée dans la réalisation de tels crème, onguent, formulation injectable, suppositoire, ou préparation orale, nasale ou pulmonaire, incorpore, avec la substance d'acide nucléique à rehausser, une quantité d'eau contenant, et de préférence saturée avec, l'oxyde nitreux, l'acide gras à longue chaîne ou son ester, et la substance d'acide nucléique formulée avec eux, et optionnellement incorpore en outre des excipients et supports supplémentaires conventionnellement utilisés dans le domaine pharmaceutique pour réaliser de telles formes de dosage.

10. Procédé selon la revendication 1 ou formulation selon la revendication 2, dans lequel la substance d'acide nucléique est sélectionnée dans le groupe consistant en A.D.N., A.R.N. hybrides A.D.N.-A.R.N., oligonucléotides et acides nucléiques synthétiques, et est de préférence un codage de séquence d'A.D.N. pour l'expression de n'importe laquelle des classes de protéines suivantes : chimiokines, chimiotactines, cytokines, enzymes, gonadotrophines, facteurs de croissance, immunoglobines, interférons, interleukines, protéines de fixation de lipides, hormones hypophysaires, inhibiteurs de protéase, protéases et somatomédines.

11. Procédé selon la revendication 1 ou composition selon la revendication 2, **caractérisé en outre en ce que** la composition comporte un anticorps peptide ou autre dérivé de peptide sélectif pour un récepteur ou antigène ou élément de fixation de protéine sélectionné sur une cellule cible, un tel peptide étant lié à au moins un des acides gras ou ses dérivés dans la composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6258789 B, German
- EP 939128773 A
- US 5633284 A
- WO 9717978 A

- US 6221377 B
- GB 1033299 A
- GB 1105919 A
- EP 0123827 A

**Non-patent literature cited in the description**

- **Goodman ; Gilman's.** The Pharmacological Basis of Therapeutics. 1990, 298-300
- **Horrobin DF.** Nutritional and medical importance of gamma-linolenic acid. *Lipid Res,* 1992, vol. 31 (2), 163-194

- **Everett DJ ; Perry CJ ; Bayliss P.** *Med Sci Res.,* 1988, vol. 16, 865-866